# EUROPEAN PATENT APPLICATION

(11) **EP 4 002 244 A1**
(43) Date of publication of application: **25.05.2022**
(21) Application number: 21200713.2
(22) Date of filing: 04.10.2021
(51) Int. Cl.: G06Q 10/08

(54) **MEDICAL SUPPLY TRACKING MANAGEMENT SYSTEM**

(30) Priority: 24.11.2020 TW 109141152
(71) Applicant: AI Biomaterial Healthtech Ltd., Hong Kong 999077 (HK)
(72) Inventor: HO, Yen-Yi, Taoyuan City 32450 (TW)
(74) Representative: Viering, Jentschura & Partner mbB Patent- und Rechtsanwälte

(57) **Abstract**

A medical supply tracking management system (900) includes a database (140), an information transmission and reception module (120) and a management module (130). The information transmission and reception module (120) is used for receiving a tag information and a manufacturing, logistics and process of use of the medical supply (1), and for sending the manufacturing, logistics and process of use to a block chain network (300) and the database (140). The management module (130) is electrically connected to the information transmission and reception module (120), and the management module (130) includes a history query module (131), wherein the history query module (131) obtains the manufacturing, logistics and process of use of the medical supply (1) corresponding to the tag information from the database (140) according to the tag information.

## Description

### BACKGROUND OF THE INVENTION

### 1. Field of the Invention

The present invention relates to a medical supply tracking management system; more particularly, the present invention relates to a medical supply tracking management system for allowing the user to check the medical data in the block chain network conveniently.

### 2. Description of the Related Art

In the process of treating diseases and injecting vaccines, various medical supplies will be used for the patient. After the pharmaceutical factory produces a common medical supply, the common medical supply is transported to the hospital by a logistics agency and administered to the patient by the medical staff of the hospital. However, there is still no system to trace the product's origin, the transport logistics, and the process of use (which is the journey from the manufacturer to the medical staff) of the medical supply. Therefore, there is a need to provide a medical supply tracking management system which can provide the information of the product's origin, the transport logistics, and the process of use of the medical supply to any user in the production terminal, logistics terminal or user terminal of the medical supply so as to control and monitor mistakes that occur in the production process, logistics process and process of use, which may cause the deterioration of drug or vaccine ingredients and cause the patient to experience allergies or other adverse reactions, and to clarify whether the responsibility lies with the manufacturer, the logistics carrier or the hospital storage and injection procedures. Thus, there is a need to provide a complete and traceable system that integrates transportation information, injection information and patient information to solve the problems of the existing technology.

### SUMMARY OF THE INVENTION

It is an object of the present invention to provide a medical supply tracking management system for allowing the user to check the medical data in the block chain network conveniently.

To achieve the abovementioned object, the medical supply tracking management system of the present invention is used for receiving a manufacturing, logistics and process of use of a medical supply sent by a third party organization such that a user can track the manufacturing, logistics and process of use, wherein the medical supply comprises an identification label and the identification label comprises a tag information. The medical supply tracking management system includes a database, an information transmission and reception module and a management module. The information transmission and reception module is used for receiving the tag information and the manufacturing, logistics and process of use of the medical supply, and for sending the manufacturing, logistics and process of use to a block chain network and the database. The management module is electrically connected to the information transmission and reception module, and the management module includes a history query module. The history query module obtains the manufacturing, logistics and process of use of the medical supply corresponding to the identification label from the database according to the tag information.

According to one embodiment of the present invention, the third party organization includes a manufacturer, a logistics provider and a user terminal of the medical supply, and the manufacturing, logistics and process of use include a manufacturing process, a logistics process and a process of use.

According to one embodiment of the present invention, the information transmission and reception module receives the manufacturing process, the logistics process and the process of use sent by the manufacturer, the logistics provider and the user terminal respectively.

According to one embodiment of the present invention, the user terminal has a tag reader, via which the user terminal obtains the tag information.

According to one embodiment of the present invention, the identification label is a radio frequency identification (RFID) label or a near-field communication (NFC) label, and the tag reader is a radio frequency identification reader or a near-field communication reader.

According to one embodiment of the present invention, the medical supply includes a medical injection.

According to one embodiment of the present invention, the user terminal is a medical institution, and the process of use is a hospital patient injection information.

### BRIEF DESCRIPTION OF THE DRAWINGS

FIG. 1 illustrates a system structure drawing of the medical supply tracking management system, the block chain network, the external computer, the pharmaceutical factory computer, the logistics computer and the medical computer in one embodiment of the present invention.
FIG. 2 illustrates an exploded view of the medical supply in one embodiment of the present invention.
FIG. 3 illustrates a schematic drawing of the medical supply and the syringe in one embodiment of the present invention.

### DETAILED DESCRIPTION OF THE PREFERRED EMBODIMENT

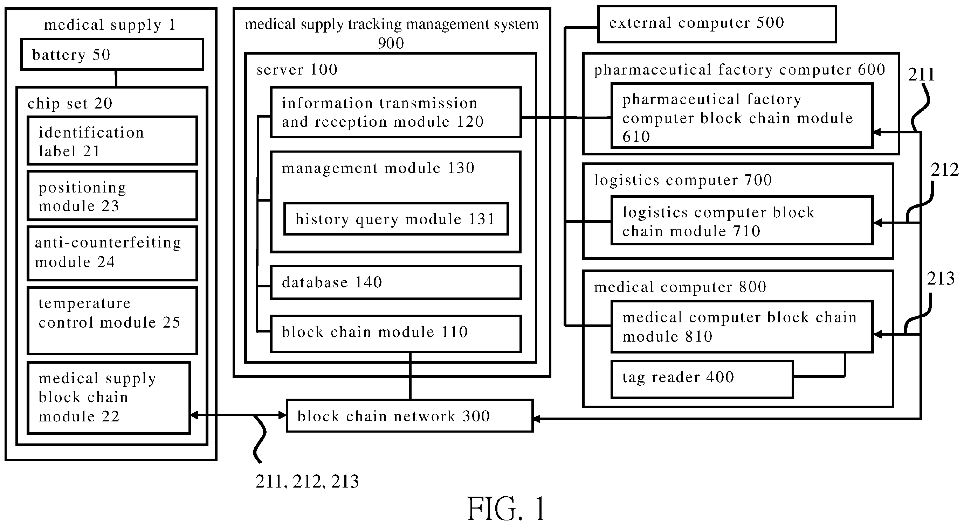

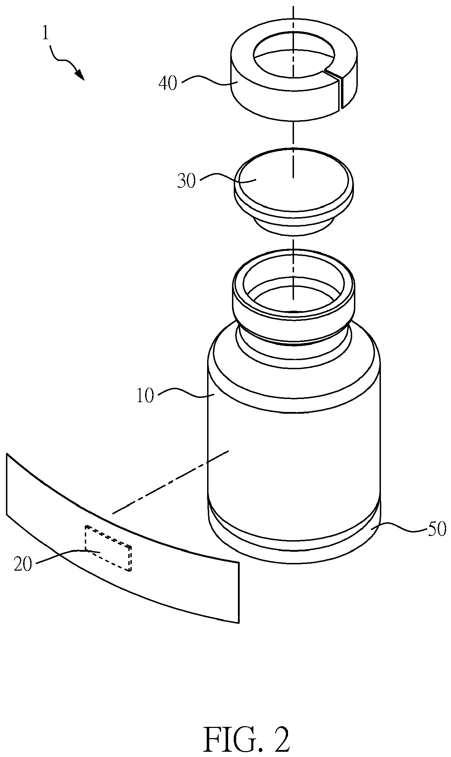

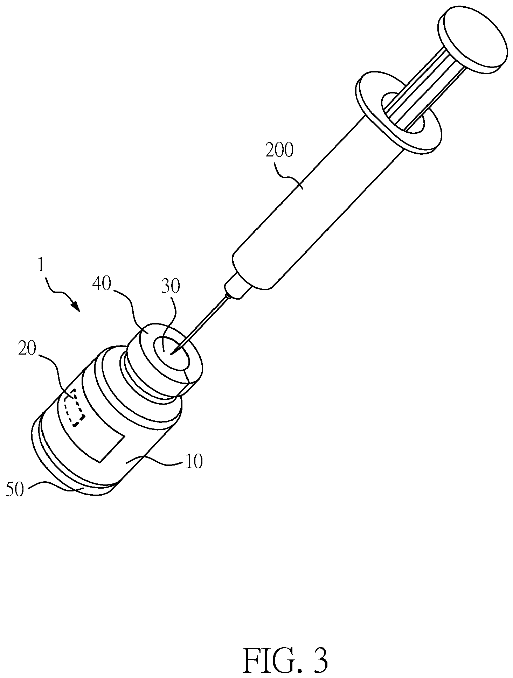

Please refer to FIG. 1 to FIG. 3, which illustrate the medical supply tracking management system in one embodiment of the present invention. FIG. 1 illustrates a system structure drawing of the medical supply tracking management system, the block chain network, the external computer, the pharmaceutical factory computer, the logistics computer and the medical computer in one embodiment of the present invention. FIG. 2 illustrates an exploded view of the medical supply in one embodiment of the present invention. FIG. 3 illustrates a schematic drawing of the medical supply and the syringe in one embodiment of the present invention.

In one embodiment of the present invention, as shown in FIG. 1, the medical supply tracking management system 900 is used for allowing any user in the third party organization to trace the manufacturing, logistics and process of use of a medical supply 1. The third party organization includes a manufacturer, a logistics provider and a user terminal. The manufacturing, logistics and process of use include a manufacturing process 211, a logistics process 212 and a process of use 213. The manufacturer is a pharmaceutical company that produces the medical supply 1. The pharmaceutical company includes a pharmaceutical factory computer 600, and the pharmaceutical factory computer 600 includes a pharmaceutical factory computer block chain module 610 for electrically connecting to the block chain network 300. The logistics provider is a logistics agency that transports the medical supply 1 from the pharmaceutical company to a hospital or pharmacy which needs the medical supply 1. The logistics agency has a logistics computer 700, and the logistics computer 700 includes a logistics computer block chain module 710 for electrically connecting to the block chain network 300. The user terminal can be a medical terminal, such as a medical institution such as a hospital or pharmacy receiving the medical supply 1. The medical institution has a medical computer 800, and the medical computer 800 includes a medical computer block chain module 810 and a tag reader 400. The medical computer block chain module 810 is used for electrically connecting to the block chain network 300, but the type of the user terminal is not limited to the abovementioned description, for the user terminal can also be a treatment terminal (such as a patient receiving the medical supply 1), a payment terminal (such as an insurance company), or a third party certifying authority (such as a backup agency).

In one embodiment of the present invention, as shown in FIG. 2 and FIG. 3, the medical supply 1 is used for containing a liquid medicine or vaccine for oral administration to a patient or for subcutaneous injection into a patent via the syringe 200. The medical supply 1 includes a bottle body 10, a chip set 20, a cover 30, a seal strip 40 and a battery 50. The bottle body 10 is used for containing the liquid medicine or vaccine. The cover 30 is used for covering the opening of the bottle body 10. The seal strip 40 is used for sealing the cover 30 on the bottle body 10 such that the user can check if the cover 30 has been opened. The battery 50 is located on the bottle body 10 for providing power to the chip set 20.

In one embodiment of the present invention, as shown in FIG. 1 and FIG. 2, the chip set 20 is a flexible chip located on the bottle body 10. The chip set 20 includes an identification label 21, a medical supply block chain module 22, a positioning module 23, an anti-counterfeiting module 24 and a temperature control module 25. The identification label 21 is a radio frequency identification (RFID) label or a near-field communication (NFC) label. The identification label 21 includes a tag information for storing a manufacturing process 211, a logistics process 212 and a process of use 213 of the medical supply 1. The manufacturing process 211 is provided by the pharmaceutical factory computer 600, and the manufacturing process 211 includes the production and manufacturing information of the medical supply 1, such as the National Drug Code (NDC) of the medical supply 1, the serial number, the batch number, the expiration date, the ingredients, and the product transaction order recorded in the supply chain. The logistics process 212 is provided by the logistics computer 700. The logistics process 212 includes the information of the temperature control record of the whole shipment, the location record of the whole shipment, the logistics vehicle number information, and the logistics officer for the medical supply 1. The process of use 213 is provided by the medical computer 800. The process of use 213 includes the hospital patient injection information, such as the medical institution where the medical supply 1 was used; the name of the pharmacy where the medical supply 1 was sold; the name of the doctor who injected the medical supply 1; the time of injecting the medical supply 1; the data of the patient who received the medical supply 1; the birthday of the patient who received the medical supply 1; the vital signs (such as blood pressure, heartbeat, respiration, body temperature or weight) of the patient who received the medical supply 1; the past allergic reactions of the patient who received the medical supply 1; the past injection record of the patient who received the medical supply 1; the family history of the patient who received the medical supply 1; the history of present illness and/or gene sequence of the patient who received the medical supply 1; or the information of the medical staff who provided the medical service.

The medical supply block chain module 22 includes a computing chip and a wireless communication chip or communication antenna, which is electrically connected to the block chain network 300, for recording the manufacturing process 211, the logistics process 212 and the process of use 213 of the medical supply 1 on the block chain network 300. Via the features that the block chain network 300 is traceable and that its records cannot easily be altered, the manufacturing process 211, the logistics process 212 and the process of use 213 of the medical supply 1 can be recorded and traced. The positioning module 23 is a global positioning system chip. The positioning module 23 is used for positioning the medical supply 1 to check the current location of the medical supply 1 and record the relationship between the delivery process and the time point throughout the entire process, to check the location and time of the delivery, and to record the transportation path. In this embodiment, the anti-counterfeiting module 24 is an anti-counterfeiting label for allowing the user to confirm the reliability and the safety of the medical supply 1. The temperature control module 25 is a chip with a temperature sensing function and is used for detecting and monitoring the temperature of the medical supply 1, whereby the user can check if the medical supply 1 is maintained at a suitable temperature to prevent detrimental effects of overheating on the drug activity of the medical supply 1.

In addition to using the medical supply block chain module 22 to access the block chain network 300 for recording the data, the pharmaceutical factory computer block chain module 610 of the pharmaceutical factory computer 600 can also access the block chain network 300 for recording the manufacturing process 211; the logistics computer block chain module 710 of the logistics computer 700 can also access the block chain network 300 for recording the logistics process 212; the medical computer block chain module 810 of the medical computer 800 can access the block chain network 300 for recording the process of use 213.

In one embodiment of the present invention, as shown in FIG. 1, the medical supply tracking management system 900 includes a server 100. The server 100 of the medical supply tracking management system 900 accesses the block chain network 300, the external computer 500, the pharmaceutical factory computer 600, the logistics computer 700 and the medical computer 800. The server 100 includes a block chain module 110, an information transmission and reception module 120, a management module 130 and a database 140. The block chain module 110 includes a computing chip and a wireless communication chip or communication antenna for electrically connecting to the block chain network 300 to obtain the manufacturing process 211, the logistics process 212 and the process of use 213 of the medical supply 1 recorded in the block chain network 300. The information transmission and reception module 120 is a network card for accessing the external computer 500, the pharmaceutical factory computer 600, the logistics computer 700 and the medical computer 800 to transfer information to one another, to receive the tag information and the manufacturing, logistics and process of use of the medical supply 1, which means that the information transmission and reception module 120 respectively receives the manufacturing process 211, logistics process 212 and process of use 213 of the medical supply 1 sent by the manufacturer, the logistics provider and the user terminal; the information transmission and reception module 120 will also transfer the received manufacturing, logistics and process of use to the block chain network 300 and the database 140. The management module 130 is a central processing processor, which is electrically connected to the block chain module 110 and the information transmission and reception module 120. The management module 130 includes a history query module 131; the history query module 131 is used for obtaining the manufacturing, logistics and process of use of the medical supply 1 corresponding to the identification label from the database 140 according to the tag information, and for transferring the manufacturing, logistics and process of use to the information transmission and reception module 120, such that the information transmission and reception module 120 can transfer the manufacturing, logistics and process of use to the external computer 500, the pharmaceutical factory computer 600, the logistics computer 700 and the medical computer 800. The database 140 is electrically connected to the block chain module 110, the information transmission and reception module 120 and the management module 130, and the database 140 is used for storing the identification label and the manufacturing, logistics and process of use.

In one embodiment of the present invention, the tag reader 400 is a radio frequency identification (RFID) reader or a near-field communication (NFC) tag reader. The tag reader 400 is electrically connected to the server 100, and the tag reader 400 is used for reading at least one of the manufacturing process 211, the logistics process 212 and the process of use 213 in the identification label 21; or the tag reader 400 can enter the manufacturing process 211, the logistics process 212 and the process of use 213 into the identification label 21, and record the manufacturing process 211, the logistics process 212 and the process of use 213 on the block chain network 300 via the medical supply block chain module 22; or the tag reader 400 can obtain the manufacturing process 211, the logistics process 212 and the process of use 213 recorded on the block chain network 300 via the medical supply block chain module 22, and enter the manufacturing process 211, the logistics process 212 and the process of use 213 recorded on the block chain network 300 via the medical supply block chain module 22 into the identification label 21. According to one embodiment of the present invention, the tag reader 400 can be directly installed in the pharmaceutical factory which produces the medical supply 1, in the logistics agency which transports the medical supply 1, or in the medical institution which uses or sells the medical supply 1, or the tag reader 400 can be installed with the management server of trace-link for tracking the information on the global pharmaceutical supply chain; the user can use the tag reader 400 to scan the identification label 21 to obtain the tag information such that medical personnel can know the manufacturing, logistics process and process of use of the medical supply 1 to ensure the safety of the patient and medication.

When the pharmaceutical company produces the medical supply 1, the staff of the pharmaceutical company can put the liquid medicine or vaccine into the bottle body 10, put the cover 30 on the bottle body 10 and apply the seal strip 40 to seal the cover 30 on the bottle body 10. Then the staff of the pharmaceutical company can use the pharmaceutical factory computer 600 to electrically connect to the tag reader 400, enter the manufacturing process 211 of the medical supply 1 into the identification label 21, record the manufacturing process 211 on the block chain network 300 via the medical supply block chain module 22, and transfer the manufacturing process 211 to the medical supply tracking management system 900 via a network. After the medical supply 1 is produced, the staff of the logistics agency can transport the medical supply 1 from the pharmaceutical company to the medical institution. During the shipping process of the logistics agency transporting the medical supply 1, the staff of the logistics agency can used the logistics computer 700 to electrically connect to the tag reader 400, enter the logistics process 212 of the medical supply 1 into the identification label 21, record the logistics process 212 on the block chain network 300 via the medical supply block chain module 22, and transfer the logistics process 212 to the medical supply tracking management system 900 via a network. After the medical supply 1 is transported to the medical institution, the staff of the medical institution can use the medical computer 800 to electrically connect to the tag reader 400, enter the process of use 213 of the medical supply 1 into the identification label 21, record the process of use 213 on the block chain network 300 via the medical supply block chain module 22, and transfer the process of use 213 to the medical supply tracking management system 900 via the network. Therefore, the block chain network 300 and the identification label 21 both record the manufacturing process 211, the logistics process 212 and the process of use 213 of the medical supply 1 completely. In addition, after the information transmission and reception module 120 of the medical supply tracking management system 900 receives the manufacturing process 211, the logistics process 212 and the process of use 213, the manufacturing process 211, the logistics process 212 and the process of use 213 will be transferred to the database 140; thus, the medical supply tracking management system 900 will also record the manufacturing process 211, the logistics process 212 and the process of use 213 of the medical supply 1 completely.

If a patient who takes the medical supply 1 has an allergic or other adverse reaction, the patient or the staff of the medical institution can use the external computer 500 to access the medical supply tracking management system 900, and use the tag reader 400 to read the tag information of the identification label 21 of the medical supply 1, and send the tag information to the medical supply tracking management system 900, to know whether any mistake occurred during the manufacturing, logistics and process of use of the medical supply 1. When the information transmission and reception module 120 receives the tag information from the external computer 500, the history query module 131 of the management module 130 will obtain the manufacturing, logistics and process of use of the medical supply 1 corresponding to the identification label from the database 140 according to the tag information; the information transmission and reception module 120 will send the manufacturing, logistics and process of use to the external computer 500. Therefore, the staff and the patient who use the external computer 500 can know the manufacturing process 211, the logistics process 212 and the process of use 213 of the medical supply 1 by checking the returned manufacturing, logistics and process of use, to furthermore understand whether the responsibility lies with the pharmaceutical company, the logistics agency or the medical institution and thereby to clarify the responsibility for medical disputes and drug relief.

However, the method of the staff of each agency for obtaining the manufacturing process 211, the logistics process 212 and the process of use 213 is not limited to the abovementioned description; for example, the staff of each agency can also use the pharmaceutical factory computer 600, the logistics computer 700 or the medical computer 800 to access the medical supply tracking management system 900 to obtain the response information, or the staff can also use the tag reader 400 to read the manufacturing process 211, the logistics process 212 and the process of use 213 stored in the identification label 21.

Via the medical supply tracking management system 900 of the present invention, the manufacturing process, the logistics process and the process of use of the medical supply can be managed and traced to improve the medication for the patient and the medical institution. The medical supply 1 and the medical supply tracking management system 900 of the present invention meet the requirement of minimal sales packaging marked on the prescription medication, improve the efficiency of the supply chain, eliminate illegal and counterfeit drugs, improve the efficiency of the recall procedure for prescription medication, and clarify the responsibility for medical disputes and drug relief. In addition, the medical supply tracking management system 900 of the present invention can collect the medical and injection records of patients worldwide who use the medical supply 1 of the present invention for use as data in a big data database of the drug and vaccine injection, provide correlation comparisons between vaccines or drug and gene sequences, upload the database to the block chain network, the pharmaceutical company (which is the manufacturing terminal), the logistics agency (which is the logistics terminal), the medical institution (which is the user terminal) and a third-party remote backup data terminal for storage and analysis of the patient's personal injections and electronic medical records to achieve the function of block chain electronic medical records.

## Claims

1. A medical supply tracking management system (900), used for receiving a manufacturing, logistics and process of use of a medical supply (1) sent by a third party organization such that a user can track the manufacturing, logistics and process of use, wherein the medical supply (1) comprises an identification label (21) and the identification label (21) comprises a tag information, **characterized in that** the the medical supply tracking management system (900) comprising:
a database (140);
an information transmission and reception module (120), for receiving the tag information and the manufacturing, logistics and process of use of the medical supply (1), and sending the manufacturing, logistics and process of use to a block chain network (300) and the database (140); and
a management module (130), electrically connected to the information transmission and reception module (120), wherein the management module (130) comprises a history query module (131), and the history query module (131) obtains the manufacturing, logistics and process of use of the medical supply (1) corresponding to the identification label (21) from the database (140) according to the tag information.

2. The medical supply tracking management system (900) as claimed in Claim 1, wherein the third party organization comprises a manufacturer, a logistics provider and a user terminal of the medical supply (1), and the manufacturing, logistics and process of use comprise a manufacturing process (211), a logistics process (212) and a process of use (213).

3. The medical supply tracking management system (900) as claimed in Claim 2, wherein the information transmission and reception module (120) receives the manufacturing process (211), the logistics process (212) and the process of use (213) sent by the manufacturer, the logistics provider and the user terminal respectively.

4. The medical supply tracking management system (900) as claimed in Claim 3, wherein the user terminal has a tag reader (400), and the user terminal obtains the tag information via the tag reader (400).

5. The medical supply tracking management system (900) as claimed in Claim 4, wherein the identification label (21) is a radio frequency identification (RFID) label or a near-field communication (NFC) label, and the tag reader (400) is a radio frequency identification reader or a near-field communication reader.

6. The medical supply tracking management system (900) as claimed in Claim 5, wherein the medical supply (1) comprises a medical injection.

7. The medical supply tracking management system (900) as claimed in Claim 6, wherein the user terminal is a medical institution, and the process of use (213) is a hospital patient injection information.
